# EUROPEAN PATENT SPECIFICATION

(11) **EP 2 188 308 B1**
(45) Date of publication and mention of the grant of the patent: **30.05.2012**
(21) Application number: 08830227.8
(22) Date of filing: 09.09.2008
(51) Int. Cl.: C07K 14/78, A61K 38/00

(54) **HIGH YIELD SECRETION OF MULTIMERIC RECOMBINANT PROTEIN**
ERTRAGREICHE ABSONDERUNG EINES MULTIMEREN REKOMBINANTEN PROTEINS
SÉCRÉTION À HAUT RENDEMENT DE PROTÉINES RECOMBINANTES MULTIMÉRIQUES

(30) Priority: 14.09.2007 EP 07116494
(43) Date of publication of application: 26.05.2010
(73) Proprietor: Fujifilm Manufacturing Europe B.V., 5047 TK Tilburg (NL)
(72) Inventor: DE BOER, Arjo Lysander, NL-5045 MD Tilburg (NL); VAN URK, Hendrik, NL-5175 CR Loon Op Zand (NL); VAN ASTEN, Peter Franciscus Theresius Maria, NL-5081 SH Hilvarenbeek (NL); BOUWSTRA, Jan Bastiaan, NL-3735 LW Bosch En Duin (NL)
(74) Representative: Morpeth, Fraser Forrest
(86) International application number: PCT/NL2008/050593
(87) International publication number: WO 2009/035323

(56) References cited:
- EP-A- 1 063 565
- EP-A- 1 238 675
- US-A- 6 150 081
- WERTEN M W T ET AL: "Secreted production of a custom-designed, highly hydrophilic gelatin in Pichia pastoris" PROTEIN ENGINEERING, OXFORD UNIVERSITY PRESS, SURREY, GB, vol. 14, no. 6, June 2001 (2001-06), pages 447-454, XP002302730 ISSN: 0269-2139 cited in the application
- WERTEN M W T ET AL: "High-yield secretion of recombinant gelatins by Pichia pastoris" YEAST, CHICHESTER, SUSSEX, GB, vol. 15, no. 11, August 1999 (1999-08), pages 1087-1096, XP002165588 ISSN: 0749-503X
- GRUSKIN E A ET AL: "A human collagen 1 (alpha1) protein helical region" GENESEQ, 2000, XP002302731
- OLSEN D ET AL: "Recombinant collagen and gelatin for drug delivery" ADVANCED DRUG DELIVERY REVIEWS, AMSTERDAM, NL, vol. 55, no. 12, 28 November 2003 (2003-11-28), pages 1547-1567, XP002368792 ISSN: 0169-409X

### SEQUENCE LISTING

<110> Fujifilm Manufacturing Europe B.V.
<120> High yield secretion of multimeric recombinant protein
<130> P6016604PCT
<150> EP 07116494.1
   <151> 2007-09-14
<160> 10
<170> PatentIn version 3.3
<210> 1
   <211> 104
   <212> PRT
   <213> Artificial
<220>
   <223> P
<400> 1
   Gly Pro Pro Gly Glu Pro Gly Asn Pro Gly Ser Pro Gly Asn Gln Gly
   1 5 10 15
   Gln Pro Gly Asn Lys Gly Ser Pro Gly Asn Pro Gly Gln Pro Gly Asn
   20 25 30
   Glu Gly Gln Pro Gly Gln Pro Gly Gln Asn Gly Gln Pro Gly Glu Pro
   35 40 45
   Gly Ser Asn Gly Pro Gln Gly Ser Gln Gly Asn Pro Gly Lys Asn Gly
   50 55 60
   Gln Pro Gly Ser Pro Gly Ser Gln Gly Ser Pro Gly Asn Gln Gly Ser
   65 70 75 80
   Pro Gly Gln Pro Gly Asn Pro Gly Gln Pro Gly Glu Gln Gly Lys Pro
   85 90 95
   Gly Asn Gln Gly Pro Ala Gly Gly
   100
<210> 2
   <211> 401
   <212> PRT
   <213> Artificial
<220>
   <223> P4
<400> 2
   Gly Pro Pro Gly Glu Pro Gly Asn Pro Gly Ser Pro Gly Asn Gln Gly
   1 5 10 15
   Gln Pro Gly Asn Lys Gly Ser Pro Gly Asn Pro Gly Gln Pro Gly Asn
   20 25 30
   Glu Gly Gln Pro Gly Gln Pro Gly Gln Asn Gly Gln Pro Gly Glu Pro
   35 40 45
   Gly Ser Asn Gly Pro Gln Gly Ser Gln Gly Asn Pro Gly Lys Asn Gly
   50 55 60
   Gln Pro Gly Ser Pro Gly Ser Gln Gly Ser Pro Gly Asn Gln Gly Ser
   65 70 75 80
   Pro Gly Gln Pro Gly Asn Pro Gly Gln Pro Gly Glu Gln Gly Lys Pro
   85 90 95
   Gly Asn Gln Gly Pro Ala Gly Glu Pro Gly Asn Pro Gly Ser Pro Gly
   100 105 110
   Asn Gln Gly Gln Pro Gly Asn Lys Gly Ser Pro Gly Asn Pro Gly Gln
   115 120 125
   Pro Gly Asn Glu Gly Gln Pro Gly Gln Pro Gly Gln Asn Gly Gln Pro
   130 135 140
   Gly Glu Pro Gly Ser Asn Gly Pro Gln Gly Ser Gln Gly Asn Pro Gly
   145 150 155 160
   Lys Asn Gly Gln Pro Gly Ser Pro Gly Ser Gln Gly Ser Pro Gly Asn
   165 170 175
   Gln Gly Ser Pro Gly Gln Pro Gly Asn Pro Gly Gln Pro Gly Glu Gln
   180 185 190
   Gly Lys Pro Gly Asn Gln Gly Pro Ala Gly Glu Pro Gly Asn Pro Gly
   195 200 205
   Ser Pro Gly Asn Gln Gly Gln Pro Gly Asn Lys Gly Ser Pro Gly Asn
   210 215 220
   Pro Gly Gln Pro Gly Asn Glu Gly Gln Pro Gly Gln Pro Gly Gln Asn
   225 230 235 240
   Gly Gln Pro Gly Glu Pro Gly Ser Asn Gly Pro Gln Gly Ser Gln Gly
   245 250 255
   Asn Pro Gly Lys Asn Gly Gln Pro Gly Ser Pro Gly Ser Gln Gly Ser
   260 265 270
   Pro Gly Asn Gln Gly Ser Pro Gly Gln Pro Gly Asn Pro Gly Gln Pro
   275 280 285
   Gly Glu Gln Gly Lys Pro Gly Asn Gln Gly Pro Ala Gly Glu Pro Gly
   290 295 300
   Asn Pro Gly Ser Pro Gly Asn Gln Gly Gln Pro Gly Asn Lys Gly Ser
   305                 310                 315                 320
   Pro Gly Asn Pro Gly Gln Pro Gly Asn Glu Gly Gln Pro Gly Gln Pro
                   325                 330                 335
   Gly Gln Asn Gly Gln Pro Gly Glu Pro Gly Ser Asn Gly Pro Gln Gly
               340                 345                 350
   Ser Gln Gly Asn Pro Gly Lys Asn Gly Gln Pro Gly Ser Pro Gly Ser
           355                 360                 365
   Gln Gly Ser Pro Gly Asn Gln Gly Ser Pro Gly Gln Pro Gly Asn Pro
       370                 375                 380
   Gly Gln Pro Gly Glu Gln Gly Lys Pro Gly Asn Gln Gly Pro Ala Gly
   385                 390                 395                 400
   Gly
<210> 3
   <211> 99
   <212> PRT
   <213> Artificial
<220>
   <223> P repeat unit
<400> 3
   Gly Glu Pro Gly Asn Pro Gly Ser Pro Gly Asn Gln Gly Gln Pro Gly
   1               5                   10                  15
   Asn Lys Gly Ser Pro Gly Asn Pro Gly Gln Pro Gly Asn Glu Gly Gln
               20                  25                  30
   Pro Gly Gln Pro Gly Gln Asn Gly Gln Pro Gly Glu Pro Gly Ser Asn
           35                  40                  45
   Gly Pro Gln Gly Ser Gln Gly Asn Pro Gly Lys Asn Gly Gln Pro Gly
       50                  55                  60
   Ser Pro Gly Ser Gln Gly Ser Pro Gly Asn Gln Gly Ser Pro Gly Gln
   65                  70                  75                  80
   Pro Gly Asn Pro Gly Gln Pro Gly Glu Gln Gly Lys Pro Gly Asn Gln
                   85                  90                  95
   Gly Pro Ala
<210> 4
   <211> 396
   <212> PRT
   <213> Artificial
<220>
   <223> P4 repeat unit
<400> 4
   Gly Glu Pro Gly Asn Pro Gly Ser Pro Gly Asn Gln Gly Gln Pro Gly
   1 5 10 15
   Asn Lys Gly Ser Pro Gly Asn Pro Gly Gln Pro Gly Asn Glu Gly Gln
   20 25 30
   Pro Gly Gln Pro Gly Gln Asn Gly Gln Pro Gly Glu Pro Gly Ser Asn
   35 40 45
   Gly Pro Gln Gly Ser Gln Gly Asn Pro Gly Lys Asn Gly Gln Pro Gly
   50 55 60
   Ser Pro Gly Ser Gln Gly Ser Pro Gly Asn Gln Gly Ser Pro Gly Gln
   65 70 75 80
   Pro Gly Asn Pro Gly Gln Pro Gly Glu Gln Gly Lys Pro Gly Asn Gln
   85 90 95
   Gly Pro Ala Gly Glu Pro Gly Asn Pro Gly Ser Pro Gly Asn Gln Gly
   100 105 110
   Gln Pro Gly Asn Lys Gly Ser Pro Gly Asn Pro Gly Gln Pro Gly Asn
   115 120 125
   Glu Gly Gln Pro Gly Gln Pro Gly Gln Asn Gly Gln Pro Gly Glu Pro
   130 135 140
   Gly Ser Asn Gly Pro Gln Gly Ser Gln Gly Asn Pro Gly Lys Asn Gly
   145 150 155 160
   Gln Pro Gly Ser Pro Gly Ser Gln Gly Ser Pro Gly Asn Gln Gly Ser
   165 170 175
   Pro Gly Gln Pro Gly Asn Pro Gly Gln Pro Gly Glu Gln Gly Lys Pro
   180 185 190
   Gly Asn Gln Gly Pro Ala Gly Glu Pro Gly Asn Pro Gly Ser Pro Gly
   195 200 205
   Asn Gln Gly Gln Pro Gly Asn Lys Gly Ser Pro Gly Asn Pro Gly Gln
   210 215 220
   Pro Gly Asn Glu Gly Gln Pro Gly Gln Pro Gly Gln Asn Gly Gln Pro
   225 230 235 240
   Gly Glu Pro Gly Ser Asn Gly Pro Gln Gly Ser Gln Gly Asn Pro Gly
                   245                 250                 255
   Lys Asn Gly Gln Pro Gly Ser Pro Gly Ser Gln Gly Ser Pro Gly Asn
               260                 265                 270
   Gln Gly Ser Pro Gly Gln Pro Gly Asn Pro Gly Gln Pro Gly Glu Gln
           275                 280                 285
   Gly Lys Pro Gly Asn Gln Gly Pro Ala Gly Glu Pro Gly Asn Pro Gly
       290                 295                 300
   Ser Pro Gly Asn Gln Gly Gln Pro Gly Asn Lys Gly Ser Pro Gly Asn
   305                 310                 315                 320
   Pro Gly Gln Pro Gly Asn Glu Gly Gln Pro Gly Gln Pro Gly Gln Asn
                   325                 330                 335
   Gly Gln Pro Gly Glu Pro Gly Ser Asn Gly Pro Gln Gly Ser Gln Gly
               340                 345                 350
   Asn Pro Gly Lys Asn Gly Gln Pro Gly Ser Pro Gly Ser Gln Gly Ser
           355                 360                 365
   Pro Gly Asn Gln Gly Ser Pro Gly Gln Pro Gly Asn Pro Gly Gln Pro
       370                 375                 380
   Gly Glu Gln Gly Lys Pro Gly Asn Gln Gly Pro Ala
   385                 390                 395
<210> 5
   <211> 792
   <212> PRT
   <213> Artificial
<220>
   <223> P 8-mer
<400> 5
   Gly Glu Pro Gly Asn Pro Gly Ser Pro Gly Asn Gln Gly Gln Pro Gly
   1               5                   10                  15
   Asn Lys Gly Ser Pro Gly Asn Pro Gly Gln Pro Gly Asn Glu Gly Gln
               20                  25                  30
   Pro Gly Gln Pro Gly Gln Asn Gly Gln Pro Gly Glu Pro Gly Ser Asn
           35                  40                  45
   Gly Pro Gln Gly Ser Gln Gly Asn Pro Gly Lys Asn Gly Gln Pro Gly
       50                  55                  60
   Ser Pro Gly Ser Gln Gly Ser Pro Gly Asn Gln Gly Ser Pro Gly Gln
   65                  70                  75                  80
   Pro Gly Asn Pro Gly Gln Pro Gly Glu Gln Gly Lys Pro Gly Asn Gln
                   85                  90                  95
   Gly Pro Ala Gly Glu Pro Gly Asn Pro Gly Ser Pro Gly Asn Gln Gly
               100                 105                 110
   Gln Pro Gly Asn Lys Gly Ser Pro Gly Asn Pro Gly Gln Pro Gly Asn
           115                 120                 125
   Glu Gly Gln Pro Gly Gln Pro Gly Gln Asn Gly Gln Pro Gly Glu Pro
       130                 135                 140
   Gly Ser Asn Gly Pro Gln Gly Ser Gln Gly Asn Pro Gly Lys Asn Gly
   145                 150                 155                 160
   Gln Pro Gly Ser Pro Gly Ser Gln Gly Ser Pro Gly Asn Gln Gly Ser
                   165                 170                 175
   Pro Gly Gln Pro Gly Asn Pro Gly Gln Pro Gly Glu Gln Gly Lys Pro
               180                 185                 190
   Gly Asn Gln Gly Pro Ala Gly Glu Pro Gly Asn Pro Gly Ser Pro Gly
           195                 200                 205
   Asn Gln Gly Gln Pro Gly Asn Lys Gly Ser Pro Gly Asn Pro Gly Gln
       210                 215                 220
   Pro Gly Asn Glu Gly Gln Pro Gly Gln Pro Gly Gln Asn Gly Gln Pro
   225                 230                 235                 240
   Gly Glu Pro Gly Ser Asn Gly Pro Gln Gly Ser Gln Gly Asn Pro Gly
                   245                 250                 255
   Lys Asn Gly Gln Pro Gly Ser Pro Gly Ser Gln Gly Ser Pro Gly Asn
               260                 265                 270
   Gln Gly Ser Pro Gly Gln Pro Gly Asn Pro Gly Gln Pro Gly Glu Gln
           275                 280                 285
   Gly Lys Pro Gly Asn Gln Gly Pro Ala Gly Glu Pro Gly Asn Pro Gly
       290                 295                 300
   Ser Pro Gly Asn Gln Gly Gln Pro Gly Asn Lys Gly Ser Pro Gly Asn
   305                 310                 315                 320
   Pro Gly Gln Pro Gly Asn Glu Gly Gln Pro Gly Gln Pro Gly Gln Asn
                   325                 330                 335
   Gly Gln Pro Gly Glu Pro Gly Ser Asn Gly Pro Gln Gly Ser Gln Gly
               340                 345                 350
   Asn Pro Gly Lys Asn Gly Gln Pro Gly Ser Pro Gly Ser Gln Gly Ser
   355 360 365
   Pro Gly Asn Gln Gly Ser Pro Gly Gln Pro Gly Asn Pro Gly Gln Pro
   370 375 380
   Gly Glu Gln Gly Lys Pro Gly Asn Gln Gly Pro Ala Gly Glu Pro Gly
   385 390 395 400
   Asn Pro Gly Ser Pro Gly Asn Gln Gly Gln Pro Gly Asn Lys Gly Ser
   405 410 415
   Pro Gly Asn Pro Gly Gln Pro Gly Asn Glu Gly Gln Pro Gly Gln Pro
   420 425 430
   Gly Gln Asn Gly Gln Pro Gly Glu Pro Gly Ser Asn Gly Pro Gln Gly
   435 440 445
   Ser Gln Gly Asn Pro Gly Lys Asn Gly Gln Pro Gly Ser Pro Gly Ser
   450 455 460
   Gln Gly Ser Pro Gly Asn Gln Gly Ser Pro Gly Gln Pro Gly Asn Pro
   465 470 475 480
   Gly Gln Pro Gly Glu Gln Gly Lys Pro Gly Asn Gln Gly Pro Ala Gly
   485 490 495
   Glu Pro Gly Asn Pro Gly Ser Pro Gly Asn Gln Gly Gln Pro Gly Asn
   500 505 510
   Lys Gly Ser Pro Gly Asn Pro Gly Gln Pro Gly Asn Glu Gly Gln Pro
   515 520 525
   Gly Gln Pro Gly Gln Asn Gly Gln Pro Gly Glu Pro Gly Ser Asn Gly
   530 535 540
   Pro Gln Gly Ser Gln Gly Asn Pro Gly Lys Asn Gly Gln Pro Gly Ser
   545 550 555 560
   Pro Gly Ser Gln Gly Ser Pro Gly Asn Gln Gly Ser Pro Gly Gln Pro
   565 570 575
   Gly Asn Pro Gly Gln Pro Gly Glu Gln Gly Lys Pro Gly Asn Gln Gly
   580 585 590
   Pro Ala Gly Glu Pro Gly Asn Pro Gly Ser Pro Gly Asn Gln Gly Gln
   595 600 605
   Pro Gly Asn Lys Gly Ser Pro Gly Asn Pro Gly Gln Pro Gly Asn Glu
   610 615 620
   Gly Gln Pro Gly Gln Pro Gly Gln Asn Gly Gln Pro Gly Glu Pro Gly
   625 630 635 640
   Ser Asn Gly Pro Gln Gly Ser Gln Gly Asn Pro Gly Lys Asn Gly Gln
   645 650 655
   Pro Gly Ser Pro Gly Ser Gln Gly Ser Pro Gly Asn Gln Gly Ser Pro
   660 665 670
   Gly Gln Pro Gly Asn Pro Gly Gln Pro Gly Glu Gln Gly Lys Pro Gly
   675 680 685
   Asn Gln Gly Pro Ala Gly Glu Pro Gly Asn Pro Gly Ser Pro Gly Asn
   690 695 700
   Gln Gly Gln Pro Gly Asn Lys Gly Ser Pro Gly Asn Pro Gly Gln Pro
   705 710 715 720
   Gly Asn Glu Gly Gln Pro Gly Gln Pro Gly Gln Asn Gly Gln Pro Gly
   725 730 735
   Glu Pro Gly Ser Asn Gly Pro Gln Gly Ser Gln Gly Asn Pro Gly Lys
   740 745 750
   Asn Gly Gln Pro Gly Ser Pro Gly Ser Gln Gly Ser Pro Gly Asn Gln
   755 760 765
   Gly Ser Pro Gly Gln Pro Gly Asn Pro Gly Gln Pro Gly Glu Gln Gly
   770 775 780
   Lys Pro Gly Asn Gln Gly Pro Ala
   785 790
<210> 6
   <211> 1188
   <212> PRT
   <213> Artificial
<220>
   <223> P 12-mer
<400> 6
   Gly Glu Pro Gly Asn Pro Gly Ser Pro Gly Asn Gln Gly Gln Pro Gly
   1 5 10 15
   Asn Lys Gly Ser Pro Gly Asn Pro Gly Gln Pro Gly Asn Glu Gly Gln
   20 25 30
   Pro Gly Gln Pro Gly Gln Asn Gly Gln Pro Gly Glu Pro Gly Ser Asn
   35 40 45
   Gly Pro Gln Gly Ser Gln Gly Asn Pro Gly Lys Asn Gly Gln Pro Gly
   50 55 60
   Ser Pro Gly Ser Gln Gly Ser Pro Gly Asn Gln Gly Ser Pro Gly Gln
   65                  70                  75                  80
   Pro Gly Asn Pro Gly Gln Pro Gly Glu Gln Gly Lys Pro Gly Asn Gln
                   85                  90                  95
   Gly Pro Ala Gly Glu Pro Gly Asn Pro Gly Ser Pro Gly Asn Gln Gly
               100                 105                 110
   Gln Pro Gly Asn Lys Gly Ser Pro Gly Asn Pro Gly Gln Pro Gly Asn
           115                 120                 125
   Glu Gly Gln Pro Gly Gln Pro Gly Gln Asn Gly Gln Pro Gly Glu Pro
       130                 135                 140
   Gly Ser Asn Gly Pro Gln Gly Ser Gln Gly Asn Pro Gly Lys Asn Gly
   145                 150                 155                 160
   Gln Pro Gly Ser Pro Gly Ser Gln Gly Ser Pro Gly Asn Gln Gly Ser
                   165                 170                 175
   Pro Gly Gln Pro Gly Asn Pro Gly Gln Pro Gly Glu Gln Gly Lys Pro
               180                 185                 190
   Gly Asn Gln Gly Pro Ala Gly Glu Pro Gly Asn Pro Gly Ser Pro Gly
           195                 200                 205
   Asn Gln Gly Gln Pro Gly Asn Lys Gly Ser Pro Gly Asn Pro Gly Gln
       210                 215                 220
   Pro Gly Asn Glu Gly Gln Pro Gly Gln Pro Gly Gln Asn Gly Gln Pro
   225                 230                 235                 240
   Gly Glu Pro Gly Ser Asn Gly Pro Gln Gly Ser Gln Gly Asn Pro Gly
                   245                 250                 255
   Lys Asn Gly Gln Pro Gly Ser Pro Gly Ser Gln Gly Ser Pro Gly Asn
               260                 265                 270
   Gln Gly Ser Pro Gly Gln Pro Gly Asn Pro Gly Gln Pro Gly Glu Gln
           275                 280                 285
   Gly Lys Pro Gly Asn Gln Gly Pro Ala Gly Glu Pro Gly Asn Pro Gly
       290                 295                 300
   Ser Pro Gly Asn Gln Gly Gln Pro Gly Asn Lys Gly Ser Pro Gly Asn
   305                 310                 315                 320
   Pro Gly Gln Pro Gly Asn Glu Gly Gln Pro Gly Gln Pro Gly Gln Asn
                   325                 330                 335
   Gly Gln Pro Gly Glu Pro Gly Ser Asn Gly Pro Gln Gly Ser Gln Gly
               340                 345                 350
   Asn Pro Gly Lys Asn Gly Gln Pro Gly Ser Pro Gly Ser Gln Gly Ser
           355                 360                 365
   Pro Gly Asn Gln Gly Ser Pro Gly Gln Pro Gly Asn Pro Gly Gln Pro
       370                 375                 380
   Gly Glu Gln Gly Lys Pro Gly Asn Gln Gly Pro Ala Gly Glu Pro Gly
   385                 390                 395                 400
   Asn Pro Gly Ser Pro Gly Asn Gln Gly Gln Pro Gly Asn Lys Gly Ser
                   405                 410                 415
   Pro Gly Asn Pro Gly Gln Pro Gly Asn Glu Gly Gln Pro Gly Gln Pro
               420                 425                 430
   Gly Gln Asn Gly Gln Pro Gly Glu Pro Gly Ser Asn Gly Pro Gln Gly
           435                 440                 445
   Ser Gln Gly Asn Pro Gly Lys Asn Gly Gln Pro Gly Ser Pro Gly Ser
       450                 455                 460
   Gln Gly Ser Pro Gly Asn Gln Gly Ser Pro Gly Gln Pro Gly Asn Pro
   465                 470                 475                 480
   Gly Gln Pro Gly Glu Gln Gly Lys Pro Gly Asn Gln Gly Pro Ala Gly
                   485                 490                 495
   Glu Pro Gly Asn Pro Gly Ser Pro Gly Asn Gln Gly Gln Pro Gly Asn
               500                 505                 510
   Lys Gly Ser Pro Gly Asn Pro Gly Gln Pro Gly Asn Glu Gly Gln Pro
           515                 520                 525
   Gly Gln Pro Gly Gln Asn Gly Gln Pro Gly Glu Pro Gly Ser Asn Gly
       530                 535                 540
   Pro Gln Gly Ser Gln Gly Asn Pro Gly Lys Asn Gly Gln Pro Gly Ser
   545                 550                 555                 560
   Pro Gly Ser Gln Gly Ser Pro Gly Asn Gln Gly Ser Pro Gly Gln Pro
                   565                 570                 575
   Gly Asn Pro Gly Gln Pro Gly Glu Gln Gly Lys Pro Gly Asn Gln Gly
               580                 585                 590
   Pro Ala Gly Glu Pro Gly Asn Pro Gly Ser Pro Gly Asn Gln Gly Gln
           595                 600                 605
   Pro Gly Asn Lys Gly Ser Pro Gly Asn Pro Gly Gln Pro Gly Asn Glu
       610                 615                 620
   Gly Gln Pro Gly Gln Pro Gly Gln Asn Gly Gln Pro Gly Glu Pro Gly
   625                 630                 635                 640
   Ser Asn Gly Pro Gln Gly Ser Gln Gly Asn Pro Gly Lys Asn Gly Gln
                   645                 650                 655
   Pro Gly Ser Pro Gly Ser Gln Gly Ser Pro Gly Asn Gln Gly Ser Pro
               660                 665                 670
   Gly Gln Pro Gly Asn Pro Gly Gln Pro Gly Glu Gln Gly Lys Pro Gly
           675                 680                 685
   Asn Gln Gly Pro Ala Gly Glu Pro Gly Asn Pro Gly Ser Pro Gly Asn
       690                 695                 700
   Gln Gly Gln Pro Gly Asn Lys Gly Ser Pro Gly Asn Pro Gly Gln Pro
   705                 710                 715                 720
   Gly Asn Glu Gly Gln Pro Gly Gln Pro Gly Gln Asn Gly Gln Pro Gly
                   725                 730                 735
   Glu Pro Gly Ser Asn Gly Pro Gln Gly Ser Gln Gly Asn Pro Gly Lys
               740                 745                 750
   Asn Gly Gln Pro Gly Ser Pro Gly Ser Gln Gly Ser Pro Gly Asn Gln
           755                 760                 765
   Gly Ser Pro Gly Gln Pro Gly Asn Pro Gly Gln Pro Gly Glu Gln Gly
       770                 775                 780
   Lys Pro Gly Asn Gln Gly Pro Ala Gly Glu Pro Gly Asn Pro Gly Ser
   785                 790                 795                 800
   Pro Gly Asn Gln Gly Gln Pro Gly Asn Lys Gly Ser Pro Gly Asn Pro
                   805                 810                 815
   Gly Gln Pro Gly Asn Glu Gly Gln Pro Gly Gln Pro Gly Gln Asn Gly
               820                 825                 830
   Gln Pro Gly Glu Pro Gly Ser Asn Gly Pro Gln Gly Ser Gln Gly Asn
           835                 840                 845
   Pro Gly Lys Asn Gly Gln Pro Gly Ser Pro Gly Ser Gln Gly Ser Pro
       850                 855                 860
   Gly Asn Gln Gly Ser Pro Gly Gln Pro Gly Asn Pro Gly Gln Pro Gly
   865                 870                 875                 880
   Glu Gln Gly Lys Pro Gly Asn Gln Gly Pro Ala Gly Glu Pro Gly Asn
                   885                 890                 895
   Pro Gly Ser Pro Gly Asn Gln Gly Gln Pro Gly Asn Lys Gly Ser Pro
               900                 905                 910
   Gly Asn Pro Gly Gln Pro Gly Asn Glu Gly Gln Pro Gly Gln Pro Gly
           915                 920                 925
   Gln Asn Gly Gln Pro Gly Glu Pro Gly Ser Asn Gly Pro Gln Gly Ser
       930                 935                 940
   Gln Gly Asn Pro Gly Lys Asn Gly Gln Pro Gly Ser Pro Gly Ser Gln
   945                 950                 955                 960
   Gly Ser Pro Gly Asn Gln Gly Ser Pro Gly Gln Pro Gly Asn Pro Gly
                   965                 970                 975
   Gln Pro Gly Glu Gln Gly Lys Pro Gly Asn Gln Gly Pro Ala Gly Glu
               980                 985                 990
   Pro Gly Asn Pro Gly Ser Pro Gly  Asn Gln Gly Gln Pro  Gly Asn Lys
           995                 1000                 1005
   Gly Ser  Pro Gly Asn Pro Gly  Gln Pro Gly Asn Glu  Gly Gln Pro
       1010                 1015                 1020
   Gly Gln  Pro Gly Gln Asn Gly  Gln Pro Gly Glu Pro  Gly Ser Asn
       1025                 1030                 1035
   Gly Pro  Gln Gly Ser Gln Gly  Asn Pro Gly Lys Asn  Gly Gln Pro
       1040                 1045                 1050
   Gly Ser  Pro Gly Ser Gln Gly  Ser Pro Gly Asn Gln  Gly Ser Pro
       1055                 1060                 1065
   Gly Gln  Pro Gly Asn Pro Gly  Gln Pro Gly Glu Gln  Gly Lys Pro
       1070                 1075                 1080
   Gly Asn  Gln Gly Pro Ala Gly  Glu Pro Gly Asn Pro  Gly Ser Pro
       1085                 1090                 1095
   Gly Asn  Gln Gly Gln Pro Gly  Asn Lys Gly Ser Pro  Gly Asn Pro
       1100                 1105                 1110
   Gly Gln  Pro Gly Asn Glu Gly  Gln Pro Gly Gln Pro  Gly Gln Asn
       1115                 1120                 1125
   Gly Gln  Pro Gly Glu Pro Gly  Ser Asn Gly Pro Gln  Gly Ser Gln
       1130                 1135                 1140
   Gly Asn  Pro Gly Lys Asn Gly  Gln Pro Gly Ser Pro  Gly Ser Gln
       1145                 1150                 1155
   Gly Ser  Pro Gly Asn Gln Gly  Ser Pro Gly Gln Pro  Gly Asn Pro
   1160 1165 1170
   Gly Gln Pro Gly Glu Gln Gly Lys Pro Gly Asn Gln Gly Pro Ala
   1175 1180 1185
<210> 7
   <211> 1584
   <212> PRT
   <213> Artificial
<220>
   <223> P 16-mer
<400> 7
   Gly Glu Pro Gly Asn Pro Gly Ser Pro Gly Asn Gln Gly Gln Pro Gly
   1 5 10 15
   Asn Lys Gly Ser Pro Gly Asn Pro Gly Gln Pro Gly Asn Glu Gly Gln
   20 25 30
   Pro Gly Gln Pro Gly Gln Asn Gly Gln Pro Gly Glu Pro Gly Ser Asn
   35 40 45
   Gly Pro Gln Gly Ser Gln Gly Asn Pro Gly Lys Asn Gly Gln Pro Gly
   50 55 60
   Ser Pro Gly Ser Gln Gly Ser Pro Gly Asn Gln Gly Ser Pro Gly Gln
   65 70 75 80
   Pro Gly Asn Pro Gly Gln Pro Gly Glu Gln Gly Lys Pro Gly Asn Gln
   85 90 95
   Gly Pro Ala Gly Glu Pro Gly Asn Pro Gly Ser Pro Gly Asn Gln Gly
   100 105 110
   Gln Pro Gly Asn Lys Gly Ser Pro Gly Asn Pro Gly Gln Pro Gly Asn
   115 120 125
   Glu Gly Gln Pro Gly Gln Pro Gly Gln Asn Gly Gln Pro Gly Glu Pro
   130 135 140
   Gly Ser Asn Gly Pro Gln Gly Ser Gln Gly Asn Pro Gly Lys Asn Gly
   145 150 155 160
   Gln Pro Gly Ser Pro Gly Ser Gln Gly Ser Pro Gly Asn Gln Gly Ser
   165 170 175
   Pro Gly Gln Pro Gly Asn Pro Gly Gln Pro Gly Glu Gln Gly Lys Pro
   180 185 190
   Gly Asn Gln Gly Pro Ala Gly Glu Pro Gly Asn Pro Gly Ser Pro Gly
   195 200 205
   Asn Gln Gly Gln Pro Gly Asn Lys Gly Ser Pro Gly Asn Pro Gly Gln
   210 215 220
   Pro Gly Asn Glu Gly Gln Pro Gly Gln Pro Gly Gln Asn Gly Gln Pro
   225 230 235 240
   Gly Glu Pro Gly Ser Asn Gly Pro Gln Gly Ser Gln Gly Asn Pro Gly
   245 250 255
   Lys Asn Gly Gln Pro Gly Ser Pro Gly Ser Gln Gly Ser Pro Gly Asn
   260 265 270
   Gln Gly Ser Pro Gly Gln Pro Gly Asn Pro Gly Gln Pro Gly Glu Gln
   275 280 285
   Gly Lys Pro Gly Asn Gln Gly Pro Ala Gly Glu Pro Gly Asn Pro Gly
   290 295 300
   Ser Pro Gly Asn Gln Gly Gln Pro Gly Asn Lys Gly Ser Pro Gly Asn
   305 310 315 320
   Pro Gly Gln Pro Gly Asn Glu Gly Gln Pro Gly Gln Pro Gly Gln Asn
   325 330 335
   Gly Gln Pro Gly Glu Pro Gly Ser Asn Gly Pro Gln Gly Ser Gln Gly
   340 345 350
   Asn Pro Gly Lys Asn Gly Gln Pro Gly Ser Pro Gly Ser Gln Gly Ser
   355 360 365
   Pro Gly Asn Gln Gly Ser Pro Gly Gln Pro Gly Asn Pro Gly Gln Pro
   370 375 380
   Gly Glu Gln Gly Lys Pro Gly Asn Gln Gly Pro Ala Gly Glu Pro Gly
   385 390 395 400
   Asn Pro Gly Ser Pro Gly Asn Gln Gly Gln Pro Gly Asn Lys Gly Ser
   405 410 415
   Pro Gly Asn Pro Gly Gln Pro Gly Asn Glu Gly Gln Pro Gly Gln Pro
   420 425 430
   Gly Gln Asn Gly Gln Pro Gly Glu Pro Gly Ser Asn Gly Pro Gln Gly
   435 440 445
   Ser Gln Gly Asn Pro Gly Lys Asn Gly Gln Pro Gly Ser Pro Gly Ser
   450 455 460
   Gln Gly Ser Pro Gly Asn Gln Gly Ser Pro Gly Gln Pro Gly Asn Pro
   465 470 475 480
   Gly Gln Pro Gly Glu Gln Gly Lys Pro Gly Asn Gln Gly Pro Ala Gly
                       485                 490                 495
   Glu Pro Gly Asn Pro Gly Ser Pro Gly Asn Gln Gly Gln Pro Gly Asn
               500                 505                 510
   Lys Gly Ser Pro Gly Asn Pro Gly Gln Pro Gly Asn Glu Gly Gln Pro
           515                 520                 525
   Gly Gln Pro Gly Gln Asn Gly Gln Pro Gly Glu Pro Gly Ser Asn Gly
       530                 535                 540
   Pro Gln Gly Ser Gln Gly Asn Pro Gly Lys Asn Gly Gln Pro Gly Ser
   545                 550                 555                 560
   Pro Gly Ser Gln Gly Ser Pro Gly Asn Gln Gly Ser Pro Gly Gln Pro
                   565                 570                 575
   Gly Asn Pro Gly Gln Pro Gly Glu Gln Gly Lys Pro Gly Asn Gln Gly
               580                 585                 590
   Pro Ala Gly Glu Pro Gly Asn Pro Gly Ser Pro Gly Asn Gln Gly Gln
           595                 600                 605
   Pro Gly Asn Lys Gly Ser Pro Gly Asn Pro Gly Gln Pro Gly Asn Glu
       610                 615                 620
   Gly Gln Pro Gly Gln Pro Gly Gln Asn Gly Gln Pro Gly Glu Pro Gly
   625                 630                 635                 640
   Ser Asn Gly Pro Gln Gly Ser Gln Gly Asn Pro Gly Lys Asn Gly Gln
                   645                 650                 655
   Pro Gly Ser Pro Gly Ser Gln Gly Ser Pro Gly Asn Gln Gly Ser Pro
               660                 665                 670
   Gly Gln Pro Gly Asn Pro Gly Gln Pro Gly Glu Gln Gly Lys Pro Gly
           675                 680                 685
   Asn Gln Gly Pro Ala Gly Glu Pro Gly Asn Pro Gly Ser Pro Gly Asn
       690                 695                 700
   Gln Gly Gln Pro Gly Asn Lys Gly Ser Pro Gly Asn Pro Gly Gln Pro
   705                 710                 715                 720
   Gly Asn Glu Gly Gln Pro Gly Gln Pro Gly Gln Asn Gly Gln Pro Gly
                   725                 730                 735
   Glu Pro Gly Ser Asn Gly Pro Gln Gly Ser Gln Gly Asn Pro Gly Lys
               740                 745                 750
   Asn Gly Gln Pro Gly Ser Pro Gly Ser Gln Gly Ser Pro Gly Asn Gln
           755                 760                 765
   Gly Ser Pro Gly Gln Pro Gly Asn Pro Gly Gln Pro Gly Glu Gln Gly
       770                 775                 780
   Lys Pro Gly Asn Gln Gly Pro Ala Gly Glu Pro Gly Asn Pro Gly Ser
   785                 790                 795                 800
   Pro Gly Asn Gln Gly Gln Pro Gly Asn Lys Gly Ser Pro Gly Asn Pro
                   805                 810                 815
   Gly Gln Pro Gly Asn Glu Gly Gln Pro Gly Gln Pro Gly Gln Asn Gly
               820                 825                 830
   Gln Pro Gly Glu Pro Gly Ser Asn Gly Pro Gln Gly Ser Gln Gly Asn
           835                 840                 845
   Pro Gly Lys Asn Gly Gln Pro Gly Ser Pro Gly Ser Gln Gly Ser Pro
       850                 855                 860
   Gly Asn Gln Gly Ser Pro Gly Gln Pro Gly Asn Pro Gly Gln Pro Gly
   865                 870                 875                 880
   Glu Gln Gly Lys Pro Gly Asn Gln Gly Pro Ala Gly Glu Pro Gly Asn
                   885                 890                 895
   Pro Gly Ser Pro Gly Asn Gln Gly Gln Pro Gly Asn Lys Gly Ser Pro
               900                 905                 910
   Gly Asn Pro Gly Gln Pro Gly Asn Glu Gly Gln Pro Gly Gln Pro Gly
           915                 920                 925
   Gln Asn Gly Gln Pro Gly Glu Pro Gly Ser Asn Gly Pro Gln Gly Ser
       930                 935                 940
   Gln Gly Asn Pro Gly Lys Asn Gly Gln Pro Gly Ser Pro Gly Ser Gln
   945                 950                 955                 960
   Gly Ser Pro Gly Asn Gln Gly Ser Pro Gly Gln Pro Gly Asn Pro Gly
                   965                 970                 975
   Gln Pro Gly Glu Gln Gly Lys Pro Gly Asn Gln Gly Pro Ala Gly Glu
               980                 985                 990
   Pro Gly Asn Pro Gly Ser Pro Gly  Asn Gln Gly Gln Pro  Gly Asn Lys
           995                 1000                 1005
   Gly Ser  Pro Gly Asn Pro Gly  Gln Pro Gly Asn Glu  Gly Gln Pro
       1010                 1015                 1020
   Gly Gln  Pro Gly Gln Asn Gly  Gln Pro Gly Glu Pro  Gly Ser Asn
       1025                 1030                 1035
   Gly Pro Gln Gly Ser Gln Gly Asn Pro Gly Lys Asn Gly Gln Pro
   1040 1045 1050
   Gly Ser Pro Gly Ser Gln Gly Ser Pro Gly Asn Gln Gly Ser Pro
   1055 1060 1065
   Gly Gln Pro Gly Asn Pro Gly Gln Pro Gly Glu Gln Gly Lys Pro
   1070 1075 1080
   Gly Asn Gln Gly Pro Ala Gly Glu Pro Gly Asn Pro Gly Ser Pro
   1085 1090 1095
   Gly Asn Gln Gly Gln Pro Gly Asn Lys Gly Ser Pro Gly Asn Pro
   1100 1105 1110
   Gly Gln Pro Gly Asn Glu Gly Gln Pro Gly Gln Pro Gly Gln Asn
   1115 1120 1125
   Gly Gln Pro Gly Glu Pro Gly Ser Asn Gly Pro Gln Gly Ser Gln
   1130 1135 1140
   Gly Asn Pro Gly Lys Asn Gly Gln Pro Gly Ser Pro Gly Ser Gln
   1145 1150 1155
   Gly Ser Pro Gly Asn Gln Gly Ser Pro Gly Gln Pro Gly Asn Pro
   1160 1165 1170
   Gly Gln Pro Gly Glu Gln Gly Lys Pro Gly Asn Gln Gly Pro Ala
   1175 1180 1185
   Gly Glu Pro Gly Asn Pro Gly Ser Pro Gly Asn Gln Gly Gln Pro
   1190 1195 1200
   Gly Asn Lys Gly Ser Pro Gly Asn Pro Gly Gln Pro Gly Asn Glu
   1205 1210 1215
   Gly Gln Pro Gly Gln Pro Gly Gln Asn Gly Gln Pro Gly Glu Pro
   1220 1225 1230
   Gly Ser Asn Gly Pro Gln Gly Ser Gln Gly Asn Pro Gly Lys Asn
   1235 1240 1245
   Gly Gln Pro Gly Ser Pro Gly Ser Gln Gly Ser Pro Gly Asn Gln
   1250 1255 1260
   Gly Ser Pro Gly Gln Pro Gly Asn Pro Gly Gln Pro Gly Glu Gln
   1265 1270 1275
   Gly Lys Pro Gly Asn Gln Gly Pro Ala Gly Glu Pro Gly Asn Pro
   1280 1285 1290
   Gly Ser  Pro Gly Asn Gln Gly  Gln Pro Gly Asn Lys  Gly Ser Pro
       1295                 1300                 1305
   Gly Asn  Pro Gly Gln Pro Gly  Asn Glu Gly Gln Pro  Gly Gln Pro
       1310                 1315                 1320
   Gly Gln  Asn Gly Gln Pro Gly  Glu Pro Gly Ser Asn  Gly Pro Gln
       1325                 1330                 1335
   Gly Ser  Gln Gly Asn Pro Gly  Lys Asn Gly Gln Pro  Gly Ser Pro
       1340                 1345                 1350
   Gly Ser  Gln Gly Ser Pro Gly  Asn Gln Gly Ser Pro  Gly Gln Pro
       1355                 1360                 1365
   Gly Asn  Pro Gly Gln Pro Gly  Glu Gln Gly Lys Pro  Gly Asn Gln
       1370                 1375                 1380
   Gly Pro  Ala Gly Glu Pro Gly  Asn Pro Gly Ser Pro  Gly Asn Gln
       1385                 1390                 1395
   Gly Gln  Pro Gly Asn Lys Gly  Ser Pro Gly Asn Pro  Gly Gln Pro
       1400                 1405                 1410
   Gly Asn  Glu Gly Gln Pro Gly  Gln Pro Gly Gln Asn  Gly Gln Pro
       1415                 1420                 1425
   Gly Glu  Pro Gly Ser Asn Gly  Pro Gln Gly Ser Gln  Gly Asn Pro
       1430                 1435                 1440
   Gly Lys  Asn Gly Gln Pro Gly  Ser Pro Gly Ser Gln  Gly Ser Pro
       1445                 1450                 1455
   Gly Asn  Gln Gly Ser Pro Gly  Gln Pro Gly Asn Pro  Gly Gln Pro
       1460                 1465                 1470
   Gly Glu  Gln Gly Lys Pro Gly  Asn Gln Gly Pro Ala  Gly Glu Pro
       1475                 1480                 1485
   Gly Asn  Pro Gly Ser Pro Gly  Asn Gln Gly Gln Pro  Gly Asn Lys
       1490                 1495                 1500
   Gly Ser  Pro Gly Asn Pro Gly  Gln Pro Gly Asn Glu  Gly Gln Pro
       1505                 1510                 1515
   Gly Gln  Pro Gly Gln Asn Gly  Gln Pro Gly Glu Pro  Gly Ser Asn
       1520                 1525                 1530
   Gly Pro  Gln Gly Ser Gln Gly  Asn Pro Gly Lys Asn  Gly Gln Pro
       1535                 1540                 1545
   Gly Ser  Pro Gly Ser Gln Gly  Ser Pro Gly Asn Gln  Gly Ser Pro
   1550 1555 1560
   Gly Gln Pro Gly Asn Pro Gly Gln Pro Gly Glu Gln Gly Lys Pro
   1565 1570 1575
   Gly Asn Gln Gly Pro Ala
   1580
<210> 8
   <211> 797
   <212> PRT
   <213> Artificial
<220>
   <223> P8
<400> 8
   Gly Pro Pro Gly Glu Pro Gly Asn Pro Gly Ser Pro Gly Asn Gln Gly
   1 5 10 15
   Gln Pro Gly Asn Lys Gly Ser Pro Gly Asn Pro Gly Gln Pro Gly Asn
   20 25 30
   Glu Gly Gln Pro Gly Gln Pro Gly Gln Asn Gly Gln Pro Gly Glu Pro
   35 40 45
   Gly Ser Asn Gly Pro Gln Gly Ser Gln Gly Asn Pro Gly Lys Asn Gly
   50 55 60
   Gln Pro Gly Ser Pro Gly Ser Gln Gly Ser Pro Gly Asn Gln Gly Ser
   65 70 75 80
   Pro Gly Gln Pro Gly Asn Pro Gly Gln Pro Gly Glu Gln Gly Lys Pro
   85 90 95
   Gly Asn Gln Gly Pro Ala Gly Glu Pro Gly Asn Pro Gly Ser Pro Gly
   100 105 110
   Asn Gln Gly Gln Pro Gly Asn Lys Gly Ser Pro Gly Asn Pro Gly Gln
   115 120 125
   Pro Gly Asn Glu Gly Gln Pro Gly Gln Pro Gly Gln Asn Gly Gln Pro
   130 135 140
   Gly Glu Pro Gly Ser Asn Gly Pro Gln Gly Ser Gln Gly Asn Pro Gly
   145 150 155 160
   Lys Asn Gly Gln Pro Gly Ser Pro Gly Ser Gln Gly Ser Pro Gly Asn
   165 170 175
   Gln Gly Ser Pro Gly Gln Pro Gly Asn Pro Gly Gln Pro Gly Glu Gln
   180 185 190
   Gly Lys Pro Gly Asn Gln Gly Pro Ala Gly Glu Pro Gly Asn Pro Gly
           195                 200                 205
   Ser Pro Gly Asn Gln Gly Gln Pro Gly Asn Lys Gly Ser Pro Gly Asn
       210                 215                 220
   Pro Gly Gln Pro Gly Asn Glu Gly Gln Pro Gly Gln Pro Gly Gln Asn
   225                 230                 235                 240
   Gly Gln Pro Gly Glu Pro Gly Ser Asn Gly Pro Gln Gly Ser Gln Gly
                   245                 250                 255
   Asn Pro Gly Lys Asn Gly Gln Pro Gly Ser Pro Gly Ser Gln Gly Ser
               260                 265                 270
   Pro Gly Asn Gln Gly Ser Pro Gly Gln Pro Gly Asn Pro Gly Gln Pro
           275                 280                 285
   Gly Glu Gln Gly Lys Pro Gly Asn Gln Gly Pro Ala Gly Glu Pro Gly
       290                 295                 300
   Asn Pro Gly Ser Pro Gly Asn Gln Gly Gln Pro Gly Asn Lys Gly Ser
   305                 310                 315                 320
   Pro Gly Asn Pro Gly Gln Pro Gly Asn Glu Gly Gln Pro Gly Gln Pro
                   325                 330                 335
   Gly Gln Asn Gly Gln Pro Gly Glu Pro Gly Ser Asn Gly Pro Gln Gly
               340                 345                 350
   Ser Gln Gly Asn Pro Gly Lys Asn Gly Gln Pro Gly Ser Pro Gly Ser
           355                 360                 365
   Gln Gly Ser Pro Gly Asn Gln Gly Ser Pro Gly Gln Pro Gly Asn Pro
       370                 375                 380
   Gly Gln Pro Gly Glu Gln Gly Lys Pro Gly Asn Gln Gly Pro Ala Gly
   385                 390                 395                 400
   Glu Pro Gly Asn Pro Gly Ser Pro Gly Asn Gln Gly Gln Pro Gly Asn
                   405                 410                 415
   Lys Gly Ser Pro Gly Asn Pro Gly Gln Pro Gly Asn Glu Gly Gln Pro
               420                 425                 430
   Gly Gln Pro Gly Gln Asn Gly Gln Pro Gly Glu Pro Gly Ser Asn Gly
           435                 440                 445
   Pro Gln Gly Ser Gln Gly Asn Pro Gly Lys Asn Gly Gln Pro Gly Ser
       450                 455                 460
   Pro Gly Ser Gln Gly Ser Pro Gly Asn Gln Gly Ser Pro Gly Gln Pro
   465 470 475 480
   Gly Asn Pro Gly Gln Pro Gly Glu Gln Gly Lys Pro Gly Asn Gln Gly
   485 490 495
   Pro Ala Gly Glu Pro Gly Asn Pro Gly Ser Pro Gly Asn Gln Gly Gln
   500 505 510
   Pro Gly Asn Lys Gly Ser Pro Gly Asn Pro Gly Gln Pro Gly Asn Glu
   515 520 525
   Gly Gln Pro Gly Gln Pro Gly Gln Asn Gly Gln Pro Gly Glu Pro Gly
   530 535 540
   Ser Asn Gly Pro Gln Gly Ser Gln Gly Asn Pro Gly Lys Asn Gly Gln
   545 550 555 560
   Pro Gly Ser Pro Gly Ser Gln Gly Ser Pro Gly Asn Gln Gly Ser Pro
   565 570 575
   Gly Gln Pro Gly Asn Pro Gly Gln Pro Gly Glu Gln Gly Lys Pro Gly
   580 585 590
   Asn Gln Gly Pro Ala Gly Glu Pro Gly Asn Pro Gly Ser Pro Gly Asn
   595 600 605
   Gln Gly Gln Pro Gly Asn Lys Gly Ser Pro Gly Asn Pro Gly Gln Pro
   610 615 620
   Gly Asn Glu Gly Gln Pro Gly Gln Pro Gly Gln Asn Gly Gln Pro Gly
   625 630 635 640
   Glu Pro Gly Ser Asn Gly Pro Gln Gly Ser Gln Gly Asn Pro Gly Lys
   645 650 655
   Asn Gly Gln Pro Gly Ser Pro Gly Ser Gln Gly Ser Pro Gly Asn Gln
   660 665 670
   Gly Ser Pro Gly Gln Pro Gly Asn Pro Gly Gln Pro Gly Glu Gln Gly
   675 680 685
   Lys Pro Gly Asn Gln Gly Pro Ala Gly Glu Pro Gly Asn Pro Gly Ser
   690 695 700
   Pro Gly Asn Gln Gly Gln Pro Gly Asn Lys Gly Ser Pro Gly Asn Pro
   705 710 715 720
   Gly Gln Pro Gly Asn Glu Gly Gln Pro Gly Gln Pro Gly Gln Asn Gly
   725 730 735
   Gln Pro Gly Glu Pro Gly Ser Asn Gly Pro Gln Gly Ser Gln Gly Asn
   740 745 750
   Pro Gly Lys Asn Gly Gln Pro Gly Ser Pro Gly Ser Gln Gly Ser Pro
   755 760 765
   Gly Asn Gln Gly Ser Pro Gly Gln Pro Gly Asn Pro Gly Gln Pro Gly
   770 775 780
   Glu Gln Gly Lys Pro Gly Asn Gln Gly Pro Ala Gly Gly
   785 790 795
<210> 9
   <211> 1193
   <212> PRT
   <213> Artificial
<220>
   <223> P12
<400> 9
   Gly Pro Pro Gly Glu Pro Gly Asn Pro Gly Ser Pro Gly Asn Gln Gly
   1 5 10 15
   Gln Pro Gly Asn Lys Gly Ser Pro Gly Asn Pro Gly Gln Pro Gly Asn
   20 25 30
   Glu Gly Gln Pro Gly Gln Pro Gly Gln Asn Gly Gln Pro Gly Glu Pro
   35 40 45
   Gly Ser Asn Gly Pro Gln Gly Ser Gln Gly Asn Pro Gly Lys Asn Gly
   50 55 60
   Gln Pro Gly Ser Pro Gly Ser Gln Gly Ser Pro Gly Asn Gln Gly Ser
   65 70 75 80
   Pro Gly Gln Pro Gly Asn Pro Gly Gln Pro Gly Glu Gln Gly Lys Pro
   85 90 95
   Gly Asn Gln Gly Pro Ala Gly Glu Pro Gly Asn Pro Gly Ser Pro Gly
   100 105 110
   Asn Gln Gly Gln Pro Gly Asn Lys Gly Ser Pro Gly Asn Pro Gly Gln
   115 120 125
   Pro Gly Asn Glu Gly Gln Pro Gly Gln Pro Gly Gln Asn Gly Gln Pro
   130 135 140
   Gly Glu Pro Gly Ser Asn Gly Pro Gln Gly Ser Gln Gly Asn Pro Gly
   145 150 155 160
   Lys Asn Gly Gln Pro Gly Ser Pro Gly Ser Gln Gly Ser Pro Gly Asn
   165 170 175
   Gln Gly Ser Pro Gly Gln Pro Gly Asn Pro Gly Gln Pro Gly Glu Gln
   180 185 190
   Gly Lys Pro Gly Asn Gln Gly Pro Ala Gly Glu Pro Gly Asn Pro Gly
   195 200 205
   Ser Pro Gly Asn Gln Gly Gln Pro Gly Asn Lys Gly Ser Pro Gly Asn
   210 215 220
   Pro Gly Gln Pro Gly Asn Glu Gly Gln Pro Gly Gln Pro Gly Gln Asn
   225 230 235 240
   Gly Gln Pro Gly Glu Pro Gly Ser Asn Gly Pro Gln Gly Ser Gln Gly
   245 250 255
   Asn Pro Gly Lys Asn Gly Gln Pro Gly Ser Pro Gly Ser Gln Gly Ser
   260 265 270
   Pro Gly Asn Gln Gly Ser Pro Gly Gln Pro Gly Asn Pro Gly Gln Pro
   275 280 285
   Gly Glu Gln Gly Lys Pro Gly Asn Gln Gly Pro Ala Gly Glu Pro Gly
   290 295 300
   Asn Pro Gly Ser Pro Gly Asn Gln Gly Gln Pro Gly Asn Lys Gly Ser
   305 310 315 320
   Pro Gly Asn Pro Gly Gln Pro Gly Asn Glu Gly Gln Pro Gly Gln Pro
   325 330 335
   Gly Gln Asn Gly Gln Pro Gly Glu Pro Gly Ser Asn Gly Pro Gln Gly
   340 345 350
   Ser Gln Gly Asn Pro Gly Lys Asn Gly Gln Pro Gly Ser Pro Gly Ser
   355 360 365
   Gln Gly Ser Pro Gly Asn Gln Gly Ser Pro Gly Gln Pro Gly Asn Pro
   370 375 380
   Gly Gln Pro Gly Glu Gln Gly Lys Pro Gly Asn Gln Gly Pro Ala Gly
   385 390 395 400
   Glu Pro Gly Asn Pro Gly Ser Pro Gly Asn Gln Gly Gln Pro Gly Asn
   405 410 415
   Lys Gly Ser Pro Gly Asn Pro Gly Gln Pro Gly Asn Glu Gly Gln Pro
   420 425 430
   Gly Gln Pro Gly Gln Asn Gly Gln Pro Gly Glu Pro Gly Ser Asn Gly
   435 440 445
   Pro Gln Gly Ser Gln Gly Asn Pro Gly Lys Asn Gly Gln Pro Gly Ser
   450 455 460
   Pro Gly Ser Gln Gly Ser Pro Gly Asn Gln Gly Ser Pro Gly Gln Pro
   465 470 475 480
   Gly Asn Pro Gly Gln Pro Gly Glu Gln Gly Lys Pro Gly Asn Gln Gly
   485 490 495
   Pro Ala Gly Glu Pro Gly Asn Pro Gly Ser Pro Gly Asn Gln Gly Gln
   500 505 510
   Pro Gly Asn Lys Gly Ser Pro Gly Asn Pro Gly Gln Pro Gly Asn Glu
   515 520 525
   Gly Gln Pro Gly Gln Pro Gly Gln Asn Gly Gln Pro Gly Glu Pro Gly
   530 535 540
   Ser Asn Gly Pro Gln Gly Ser Gln Gly Asn Pro Gly Lys Asn Gly Gln
   545 550 555 560
   Pro Gly Ser Pro Gly Ser Gln Gly Ser Pro Gly Asn Gln Gly Ser Pro
   565 570 575
   Gly Gln Pro Gly Asn Pro Gly Gln Pro Gly Glu Gln Gly Lys Pro Gly
   580 585 590
   Asn Gln Gly Pro Ala Gly Glu Pro Gly Asn Pro Gly Ser Pro Gly Asn
   595 600 605
   Gln Gly Gln Pro Gly Asn Lys Gly Ser Pro Gly Asn Pro Gly Gln Pro
   610 615 620
   Gly Asn Glu Gly Gln Pro Gly Gln Pro Gly Gln Asn Gly Gln Pro Gly
   625 630 635 640
   Glu Pro Gly Ser Asn Gly Pro Gln Gly Ser Gln Gly Asn Pro Gly Lys
   645 650 655
   Asn Gly Gln Pro Gly Ser Pro Gly Ser Gln Gly Ser Pro Gly Asn Gln
   660 665 670
   Gly Ser Pro Gly Gln Pro Gly Asn Pro Gly Gln Pro Gly Glu Gln Gly
   675 680 685
   Lys Pro Gly Asn Gln Gly Pro Ala Gly Glu Pro Gly Asn Pro Gly Ser
   690 695 700
   Pro Gly Asn Gln Gly Gln Pro Gly Asn Lys Gly Ser Pro Gly Asn Pro
   705 710 715 720
   Gly Gln Pro Gly Asn Glu Gly Gln Pro Gly Gln Pro Gly Gln Asn Gly
   725 730 735
   Gln Pro Gly Glu Pro Gly Ser Asn Gly Pro Gln Gly Ser Gln Gly Asn
               740                 745                 750
   Pro Gly Lys Asn Gly Gln Pro Gly Ser Pro Gly Ser Gln Gly Ser Pro
           755                 760                 765
   Gly Asn Gln Gly Ser Pro Gly Gln Pro Gly Asn Pro Gly Gln Pro Gly
       770                 775                 780
   Glu Gln Gly Lys Pro Gly Asn Gln Gly Pro Ala Gly Glu Pro Gly Asn
   785                 790                 795                 800
   Pro Gly Ser Pro Gly Asn Gln Gly Gln Pro Gly Asn Lys Gly Ser Pro
                   805                 810                 815
   Gly Asn Pro Gly Gln Pro Gly Asn Glu Gly Gln Pro Gly Gln Pro Gly
               820                 825                 830
   Gln Asn Gly Gln Pro Gly Glu Pro Gly Ser Asn Gly Pro Gln Gly Ser
           835                 840                 845
   Gln Gly Asn Pro Gly Lys Asn Gly Gln Pro Gly Ser Pro Gly Ser Gln
       850                 855                 860
   Gly Ser Pro Gly Asn Gln Gly Ser Pro Gly Gln Pro Gly Asn Pro Gly
   865                 870                 875                 880
   Gln Pro Gly Glu Gln Gly Lys Pro Gly Asn Gln Gly Pro Ala Gly Glu
                   885                 890                 895
   Pro Gly Asn Pro Gly Ser Pro Gly Asn Gln Gly Gln Pro Gly Asn Lys
               900                 905                 910
   Gly Ser Pro Gly Asn Pro Gly Gln Pro Gly Asn Glu Gly Gln Pro Gly
           915                 920                 925
   Gln Pro Gly Gln Asn Gly Gln Pro Gly Glu Pro Gly Ser Asn Gly Pro
       930                 935                 940
   Gln Gly Ser Gln Gly Asn Pro Gly Lys Asn Gly Gln Pro Gly Ser Pro
   945                 950                 955                 960
   Gly Ser Gln Gly Ser Pro Gly Asn Gln Gly Ser Pro Gly Gln Pro Gly
                   965                 970                 975
   Asn Pro Gly Gln Pro Gly Glu Gln Gly Lys Pro Gly Asn Gln Gly Pro
               980                 985                 990
   Ala Gly Glu Pro Gly Asn Pro Gly  Ser Pro Gly Asn Gln  Gly Gln Pro
           995                 1000                 1005
   Gly Asn Lys Gly Ser Pro Gly Asn Pro Gly Gln Pro Gly Asn Glu
   1010 1015 1020
   Gly Gln Pro Gly Gln Pro Gly Gln Asn Gly Gln Pro Gly Glu Pro
   1025 1030 1035
   Gly Ser Asn Gly Pro Gln Gly Ser Gln Gly Asn Pro Gly Lys Asn
   1040 1045 1050
   Gly Gln Pro Gly Ser Pro Gly Ser Gln Gly Ser Pro Gly Asn Gln
   1055 1060 1065
   Gly Ser Pro Gly Gln Pro Gly Asn Pro Gly Gln Pro Gly Glu Gln
   1070 1075 1080
   Gly Lys Pro Gly Asn Gln Gly Pro Ala Gly Glu Pro Gly Asn Pro
   1085 1090 1095
   Gly Ser Pro Gly Asn Gln Gly Gln Pro Gly Asn Lys Gly Ser Pro
   1100 1105 1110
   Gly Asn Pro Gly Gln Pro Gly Asn Glu Gly Gln Pro Gly Gln Pro
   1115 1120 1125
   Gly Gln Asn Gly Gln Pro Gly Glu Pro Gly Ser Asn Gly Pro Gln
   1130 1135 1140
   Gly Ser Gln Gly Asn Pro Gly Lys Asn Gly Gln Pro Gly Ser Pro
   1145 1150 1155
   Gly Ser Gln Gly Ser Pro Gly Asn Gln Gly Ser Pro Gly Gln Pro
   1160 1165 1170
   Gly Asn Pro Gly Gln Pro Gly Glu Gln Gly Lys Pro Gly Asn Gln
   1175 1180 1185
   Gly Pro Ala Gly Gly
   1190
<210> 10
   <211> 1589
   <212> PRT
   <213> Artificial
<220>
   <223> P16
<400> 10
   Gly Pro Pro Gly Glu Pro Gly Asn Pro Gly Ser Pro Gly Asn Gln Gly
   1               5                   10                  15
   Gln Pro Gly Asn Lys Gly Ser Pro Gly Asn Pro Gly Gln Pro Gly Asn
               20                  25                  30
   Glu Gly Gln Pro Gly Gln Pro Gly Gln Asn Gly Gln Pro Gly Glu Pro
           35                  40                  45
   Gly Ser Asn Gly Pro Gln Gly Ser Gln Gly Asn Pro Gly Lys Asn Gly
       50                  55                  60
   Gln Pro Gly Ser Pro Gly Ser Gln Gly Ser Pro Gly Asn Gln Gly Ser
   65                  70                  75                  80
   Pro Gly Gln Pro Gly Asn Pro Gly Gln Pro Gly Glu Gln Gly Lys Pro
                   85                  90                  95
   Gly Asn Gln Gly Pro Ala Gly Glu Pro Gly Asn Pro Gly Ser Pro Gly
               100                 105                 110
   Asn Gln Gly Gln Pro Gly Asn Lys Gly Ser Pro Gly Asn Pro Gly Gln
           115                 120                 125
   Pro Gly Asn Glu Gly Gln Pro Gly Gln Pro Gly Gln Asn Gly Gln Pro
       130                 135                 140
   Gly Glu Pro Gly Ser Asn Gly Pro Gln Gly Ser Gln Gly Asn Pro Gly
   145                 150                 155                 160
   Lys Asn Gly Gln Pro Gly Ser Pro Gly Ser Gln Gly Ser Pro Gly Asn
                   165                 170                 175
   Gln Gly Ser Pro Gly Gln Pro Gly Asn Pro Gly Gln Pro Gly Glu Gln
               180                 185                 190
   Gly Lys Pro Gly Asn Gln Gly Pro Ala Gly Glu Pro Gly Asn Pro Gly
           195                 200                 205
   Ser Pro Gly Asn Gln Gly Gln Pro Gly Asn Lys Gly Ser Pro Gly Asn
       210                 215                 220
   Pro Gly Gln Pro Gly Asn Glu Gly Gln Pro Gly Gln Pro Gly Gln Asn
   225                 230                 235                 240
   Gly Gln Pro Gly Glu Pro Gly Ser Asn Gly Pro Gln Gly Ser Gln Gly
                   245                 250                 255
   Asn Pro Gly Lys Asn Gly Gln Pro Gly Ser Pro Gly Ser Gln Gly Ser
               260                 265                 270
   Pro Gly Asn Gln Gly Ser Pro Gly Gln Pro Gly Asn Pro Gly Gln Pro
           275                 280                 285
   Gly Glu Gln Gly Lys Pro Gly Asn Gln Gly Pro Ala Gly Glu Pro Gly
       290                 295                 300
   Asn Pro Gly Ser Pro Gly Asn Gln Gly Gln Pro Gly Asn Lys Gly Ser
   305 310 315 320
   Pro Gly Asn Pro Gly Gln Pro Gly Asn Glu Gly Gln Pro Gly Gln Pro
   325 330 335
   Gly Gln Asn Gly Gln Pro Gly Glu Pro Gly Ser Asn Gly Pro Gln Gly
   340 345 350
   Ser Gln Gly Asn Pro Gly Lys Asn Gly Gln Pro Gly Ser Pro Gly Ser
   355 360 365
   Gln Gly Ser Pro Gly Asn Gln Gly Ser Pro Gly Gln Pro Gly Asn Pro
   370 375 380
   Gly Gln Pro Gly Glu Gln Gly Lys Pro Gly Asn Gln Gly Pro Ala Gly
   385 390 395 400
   Glu Pro Gly Asn Pro Gly Ser Pro Gly Asn Gln Gly Gln Pro Gly Asn
   405 410 415
   Lys Gly Ser Pro Gly Asn Pro Gly Gln Pro Gly Asn Glu Gly Gln Pro
   420 425 430
   Gly Gln Pro Gly Gln Asn Gly Gln Pro Gly Glu Pro Gly Ser Asn Gly
   435 440 445
   Pro Gln Gly Ser Gln Gly Asn Pro Gly Lys Asn Gly Gln Pro Gly Ser
   450 455 460
   Pro Gly Ser Gln Gly Ser Pro Gly Asn Gln Gly Ser Pro Gly Gln Pro
   465 470 475 480
   Gly Asn Pro Gly Gln Pro Gly Glu Gln Gly Lys Pro Gly Asn Gln Gly
   485 490 495
   Pro Ala Gly Glu Pro Gly Asn Pro Gly Ser Pro Gly Asn Gln Gly Gln
   500 505 510
   Pro Gly Asn Lys Gly Ser Pro Gly Asn Pro Gly Gln Pro Gly Asn Glu
   515 520 525
   Gly Gln Pro Gly Gln Pro Gly Gln Asn Gly Gln Pro Gly Glu Pro Gly
   530 535 540
   Ser Asn Gly Pro Gln Gly Ser Gln Gly Asn Pro Gly Lys Asn Gly Gln
   545 550 555 560
   Pro Gly Ser Pro Gly Ser Gln Gly Ser Pro Gly Asn Gln Gly Ser Pro
   565 570 575
   Gly Gln Pro Gly Asn Pro Gly Gln Pro Gly Glu Gln Gly Lys Pro Gly
   580 585 590
   Asn Gln Gly Pro Ala Gly Glu Pro Gly Asn Pro Gly Ser Pro Gly Asn
   595 600 605
   Gln Gly Gln Pro Gly Asn Lys Gly Ser Pro Gly Asn Pro Gly Gln Pro
   610 615 620
   Gly Asn Glu Gly Gln Pro Gly Gln Pro Gly Gln Asn Gly Gln Pro Gly
   625 630 635 640
   Glu Pro Gly Ser Asn Gly Pro Gln Gly Ser Gln Gly Asn Pro Gly Lys
   645 650 655
   Asn Gly Gln Pro Gly Ser Pro Gly Ser Gln Gly Ser Pro Gly Asn Gln
   660 665 670
   Gly Ser Pro Gly Gln Pro Gly Asn Pro Gly Gln Pro Gly Glu Gln Gly
   675 680 685
   Lys Pro Gly Asn Gln Gly Pro Ala Gly Glu Pro Gly Asn Pro Gly Ser
   690 695 700
   Pro Gly Asn Gln Gly Gln Pro Gly Asn Lys Gly Ser Pro Gly Asn Pro
   705 710 715 720
   Gly Gln Pro Gly Asn Glu Gly Gln Pro Gly Gln Pro Gly Gln Asn Gly
   725 730 735
   Gln Pro Gly Glu Pro Gly Ser Asn Gly Pro Gln Gly Ser Gln Gly Asn
   740 745 750
   Pro Gly Lys Asn Gly Gln Pro Gly Ser Pro Gly Ser Gln Gly Ser Pro
   755 760 765
   Gly Asn Gln Gly Ser Pro Gly Gln Pro Gly Asn Pro Gly Gln Pro Gly
   770 775 780
   Glu Gln Gly Lys Pro Gly Asn Gln Gly Pro Ala Gly Glu Pro Gly Asn
   785 790 795 800
   Pro Gly Ser Pro Gly Asn Gln Gly Gln Pro Gly Asn Lys Gly Ser Pro
   805 810 815
   Gly Asn Pro Gly Gln Pro Gly Asn Glu Gly Gln Pro Gly Gln Pro Gly
   820 825 830
   Gln Asn Gly Gln Pro Gly Glu Pro Gly Ser Asn Gly Pro Gln Gly Ser
   835 840 845
   Gln Gly Asn Pro Gly Lys Asn Gly Gln Pro Gly Ser Pro Gly Ser Gln
   850 855 860
   Gly Ser Pro Gly Asn Gln Gly Ser Pro Gly Gln Pro Gly Asn Pro Gly
   865                 870                 875                 880
   Gln Pro Gly Glu Gln Gly Lys Pro Gly Asn Gln Gly Pro Ala Gly Glu
                   885                 890                 895
   Pro Gly Asn Pro Gly Ser Pro Gly Asn Gln Gly Gln Pro Gly Asn Lys
               900                 905                 910
   Gly Ser Pro Gly Asn Pro Gly Gln Pro Gly Asn Glu Gly Gln Pro Gly
           915                 920                 925
   Gln Pro Gly Gln Asn Gly Gln Pro Gly Glu Pro Gly Ser Asn Gly Pro
       930                 935                 940
   Gln Gly Ser Gln Gly Asn Pro Gly Lys Asn Gly Gln Pro Gly Ser Pro
   945                 950                 955                 960
   Gly Ser Gln Gly Ser Pro Gly Asn Gln Gly Ser Pro Gly Gln Pro Gly
                   965                 970                 975
   Asn Pro Gly Gln Pro Gly Glu Gln Gly Lys Pro Gly Asn Gln Gly Pro
               980                 985                 990
   Ala Gly Glu Pro Gly Asn Pro Gly Ser Pro Gly Asn Gln Gly Gln Pro
           995                 1000                1005
   Gly Asn Lys Gly Ser Pro Gly Asn Pro Gly Gln Pro Gly Asn Glu
       1010                1015                1020
   Gly Gln Pro Gly Gln Pro Gly Gln Asn Gly Gln Pro Gly Glu Pro
       1025                1030                1035
   Gly Ser Asn Gly Pro Gln Gly Ser Gln Gly Asn Pro Gly Lys Asn
       1040                1045                1050
   Gly Gln Pro Gly Ser Pro Gly Ser Gln Gly Ser Pro Gly Asn Gln
       1055                1060                1065
   Gly Ser Pro Gly Gln Pro Gly Asn Pro Gly Gln Pro Gly Glu Gln
       1070                1075                1080
   Gly Lys Pro Gly Asn Gln Gly Pro Ala Gly Glu Pro Gly Asn Pro
       1085                1090                1095
   Gly Ser Pro Gly Asn Gln Gly Gln Pro Gly Asn Lys Gly Ser Pro
       1100                1105                1110
   Gly Asn Pro Gly Gln Pro Gly Asn Glu Gly Gln Pro Gly Gln Pro
       1115                1120                1125
   Gly Gln Asn Gly Gln Pro Gly Glu Pro Gly Ser Asn Gly Pro Gln
   1130 1135 1140
   Gly Ser Gln Gly Asn Pro Gly Lys Asn Gly Gln Pro Gly Ser Pro
   1145 1150 1155
   Gly Ser Gln Gly Ser Pro Gly Asn Gln Gly Ser Pro Gly Gln Pro
   1160 1165 1170
   Gly Asn Pro Gly Gln Pro Gly Glu Gln Gly Lys Pro Gly Asn Gln
   1175 1180 1185
   Gly Pro Ala Gly Glu Pro Gly Asn Pro Gly Ser Pro Gly Asn Gln
   1190 1195 1200
   Gly Gln Pro Gly Asn Lys Gly Ser Pro Gly Asn Pro Gly Gln Pro
   1205 1210 1215
   Gly Asn Glu Gly Gln Pro Gly Gln Pro Gly Gln Asn Gly Gln Pro
   1220 1225 1230
   Gly Glu Pro Gly Ser Asn Gly Pro Gln Gly Ser Gln Gly Asn Pro
   1235 1240 1245
   Gly Lys Asn Gly Gln Pro Gly Ser Pro Gly Ser Gln Gly Ser Pro
   1250 1255 1260
   Gly Asn Gln Gly Ser Pro Gly Gln Pro Gly Asn Pro Gly Gln Pro
   1265 1270 1275
   Gly Glu Gln Gly Lys Pro Gly Asn Gln Gly Pro Ala Gly Glu Pro
   1280 1285 1290
   Gly Asn Pro Gly Ser Pro Gly Asn Gln Gly Gln Pro Gly Asn Lys
   1295 1300 1305
   Gly Ser Pro Gly Asn Pro Gly Gln Pro Gly Asn Glu Gly Gln Pro
   1310 1315 1320
   Gly Gln Pro Gly Gln Asn Gly Gln Pro Gly Glu Pro Gly Ser Asn
   1325 1330 1335
   Gly Pro Gln Gly Ser Gln Gly Asn Pro Gly Lys Asn Gly Gln Pro
   1340 1345 1350
   Gly Ser Pro Gly Ser Gln Gly Ser Pro Gly Asn Gln Gly Ser Pro
   1355 1360 1365
   Gly Gln Pro Gly Asn Pro Gly Gln Pro Gly Glu Gln Gly Lys Pro
   1370 1375 1380
   Gly Asn  Gln Gly Pro Ala Gly  Glu Pro Gly Asn Pro  Gly Ser Pro
       1385                 1390                 1395
   Gly Asn  Gln Gly Gln Pro Gly  Asn Lys Gly Ser Pro  Gly Asn Pro
       1400                 1405                 1410
   Gly Gln  Pro Gly Asn Glu Gly  Gln Pro Gly Gln Pro  Gly Gln Asn
       1415                 1420                 1425
   Gly Gln  Pro Gly Glu Pro Gly  Ser Asn Gly Pro Gln  Gly Ser Gln
       1430                 1435                 1440
   Gly Asn  Pro Gly Lys Asn Gly  Gln Pro Gly Ser Pro  Gly Ser Gln
       1445                 1450                 1455
   Gly Ser  Pro Gly Asn Gln Gly  Ser Pro Gly Gln Pro  Gly Asn Pro
       1460                 1465                 1470
   Gly Gln  Pro Gly Glu Gln Gly  Lys Pro Gly Asn Gln  Gly Pro Ala
       1475                 1480                 1485
   Gly Glu  Pro Gly Asn Pro Gly  Ser Pro Gly Asn Gln  Gly Gln Pro
       1490                 1495                 1500
   Gly Asn  Lys Gly Ser Pro Gly  Asn Pro Gly Gln Pro  Gly Asn Glu
       1505                 1510                 1515
   Gly Gln  Pro Gly Gln Pro Gly  Gln Asn Gly Gln Pro  Gly Glu Pro
       1520                 1525                 1530
   Gly Ser  Asn Gly Pro Gln Gly  Ser Gln Gly Asn Pro  Gly Lys Asn
       1535                 1540                 1545
   Gly Gln  Pro Gly Ser Pro Gly  Ser Gln Gly Ser Pro  Gly Asn Gln
       1550                 1555                 1560
   Gly Ser  Pro Gly Gln Pro Gly  Asn Pro Gly Gln Pro  Gly Glu Gln
       1565                 1570                 1575
   Gly Lys  Pro Gly Asn Gln Gly  Pro Ala Gly Gly
       1580                 1585

## Description

### FIELD OF THE INVENTION

The present invention relates to the field of gene expression systems and the production of recombinant proteins or polypeptides. Provided are methods for producing high yields of high molecular weight gelatins (40 kDa and larger), as well as the gelatins as such and compositions comprising one or more of these or consisting of one or more of these.

### BACKGROUND OF THE INVENTION

Werten et al. (2001, Protein Engineering 14:447-454) describe the production of a recombinant polar (hydrophilic) gelatin-like polypeptide in *Pichia pastoris.* Both a monomeric unit (P) (SEQ ID NO: 1) and a tetrameric unit (P4) (SEQ ID NO 2) having four repeat units (P repeat units) (SEQ ID NO:3) are expressed. The yield of the tetrameric (P4) was found to be 3-6 g/l of clarified broth. The P4 polypeptide was non-hydroxylated and had an open structure (i.e. did not form triple helices at 4°C and was essentially non-gelling). Also, the high polarity accounted for negligible surface activity in water at concentrations of up to 5% (w/v) as determined by tensiometry.

EP 0926543 describes the production of non-hydroxylated, recombinant mouse type I (Col1A1, 28kDa and 53kDa) and rat type III (Col3A1, 21 kDa) collagen-like polypeptides in *Pichia pastoris* at a yield of 2-3 g/l for single copy tranformants, with up to 14.8 g/liter clarified broth of multicopy transformants (Werten et al. 1999, Yeast 15, 1087-1096). The polypeptides used were fragments of natural Col1A1 and Col1A3, whereby the fragments were part of the triple helix domain, comprising Gly-Xaa-Yaa triplets.

Many problems exist with instability of highly repetitive synthetic genes (Cappello, 1990, Trends Biotechnol. 8, 309-311). Native gelatin sequences may be more stable than synthetic sequences, due to their higher variability in amino acid sequence.

There remains a need for producing high yields of recombinant, synthetic collagen-like proteins or polypeptides, especially synthetic sequences and/or non-gelling (non-hydroxylated) sequences. High molecular weight sequences (such as polypeptides of calculated molecular weights of 70kDa or more) are particularly difficult to produce at high yields, as degradation problems are more problematic than for low molecular weight polypeptides.

### SUMMARY OF THE INVENTION

In the search for further improvements of the yields at the production of recombinant gelatins which might be suitable for various applications, the present inventors surprisingly found that upon trying to recombinantly produce gelatins with high molecular weight, the yield of the polypeptide obtained was unexpectedly high, in fact even better than for similar, yet smaller polypeptides. A multimer polypeptide was identified consisting of or comprising at least 5 consecutive repeat units of a monomer polypeptide unit, wherein said monomer polypeptide unit consists of or comprises the amino acid sequence SEQ ID NO:3. In one embodiment of the invention, the recombinant gelatins are provided, as well as pharmaceutical or nutraceutical compositions or cell supports comprising the recombinant gelatins. Also methods for using the recombinant gelatins and/or the cell supports or controlled release compositions for cell adhesion related medical applications are provided.

### GENERAL DEFINITIONS

"Gelatin" and "gelatin-like" and "collagen" and "collagen-like" proteins or polypeptides are used herein interchangeably to refer to amino acid chains comprising or consisting of Gly-Xaa-Yaa (GXY) repeats. Also, the terms "gelatin", "protein", "peptide" and "polypeptide" are used interchangeably herein.

"High molecular weight" refers herein to polypeptides of at least about 40 kDa calculated molecular weight, such as polypeptides of equal to or above about 50, 60 and in particular of equal to or above about 70, 80, 90, 100, 110, 120, 130, 140, 150, 200, 250 up to 300 kDa, or more.

The term "substantially identical", "substantial identity" or "essentially similar" or "essential similarity" means that two polypeptide, when aligned pairwise using the Smith-Waterman algorithm using default parameters, comprise at least 70%, 72%, 74%, 75%, 76%, 77% or 78%, preferably at least 80%, more preferably at least 85%, 90%, 95%, 98%, 99% or more amino acid sequence identity. More preferably, the polypeptides comprise said amino acid sequence identity while having no more than 3 gaps, preferably no more than 2 gaps, even more preferably no more than 1 gap and most preferably 0 gaps in the alignment. Sequence alignments and scores for percentage sequence identity may be determined using computer programs, such as the GCG Wisconsin Package, Version 10.3, available from Accelrys Inc., 9685 Scranton Road, San Diego, CA 92121-3752 USA or using in EmbossWIN (e.g. version 2.10.0). For comparing sequence identity between two sequences, it is preferred that local alignment algorithms are used, such as the Smith Waterman algorithm (Smith TF, Waterman MS (1981) J. Mol. Biol 147(1);195-7), used e.g. in the EmbossWIN program "water". Default parameters are gap opening penalty 10.0 and gap extension penalty 0.5, using the Blosum62 substitution matrix for proteins (Henikoff & Henikoff, 1992, PNAS 89, 915-919).

The term "comprising" is to be interpreted as specifying the presence of the stated parts, steps or components, but does not exclude the presence of one or more additional parts, steps or components.

In addition, reference to an element by the indefinite article "a" or "an" does not exclude the possibility that more than one of the element is present, unless the context clearly requires that there be one and only one of the elements. The indefinite article "a" or "an" thus usually means "at least one".

"Monomer" refers to a polypeptide unit which can be used to generate a "multimer" by repeating the unit in a linear fashion to generate a longer polypeptide. The monomer units are preferably repeated without intervening amino acids, although optionally 1, 2, 3, 4 or 5 linking amino acids may be present between monomer units. Polypeptide with (SEQ ID NO: 3) herein is an example of a monomer. Polypeptide with (SEQ ID NO: 4) herein is a "repeat with molecular weight below 70 kDa", in particular the "repeat" is a tetramer, of the repeat unit of monomer "P" (SEQ ID NO: 3). The repeat unit of Polypeptide "P4" (SEQ ID NO: 4) may be repeated 2, 3, 4 times or more to form multimers of 8 P repeat units (8-mer) (SEQ ID NO: 5), 12 P repeat units (12-mer) (SEQ ID NO: 6), 16 P repeat units (16-mer) (SEQ ID NO: 7), etc.

"Host" or "host organism" or "recombinant host cell" refers herein to the microorganism into which the nucleic acid sequence encoding the polypeptide according to the invention is introduced. Preferred hosts are yeasts of the genus Pichia (preferably *Pichia pastoris,* Hansenula (preferably *Hansenula polymorpha*), Axula (preferably *Axula adeninivorans*)

### DETAILED DESCRIPTION

### Polypeptides and nucleic acid sequences according to the invention

In one aspect of the invention gelatin-like polypeptides are provided, which have a high molecular weight. Gelatin-like polypeptides comprise the monomer unit (SEQ ID NO:3), which is preferably repeated at least 5, 6, 7, 8, 9, 10, 11, 12, 13, 14, 15, 16, 17, 18, 19 or 20 times to form a high molecular weight polypeptide. In a preferred embodiment the monomer unit is repeated with an even number of repeats, i.e. the high molecular weight polypeptide comprises at least 8, 10, 12, 14, 16, 18, 20, 24, 28 or 32 times the monomer unit.

Herein, the polypeptides according to the invention are preferably essentially free of posttranslational modifications by prolyl-4-hydroxylase (P4H) enzymes, as they are produced in recombinant host cells, such a the methylotrophic yeast *Pichia,* into which no heterologous genes encoding a functional P4H enzyme have been introduced. Thus, in one aspect of the invention, the monomer and multimer are free of hydroxyproline and free of triple helix structure characteristic of natural collagen. "Free of hydroxyproline" refers herein to gelatin-like polypeptides that are in essence free of hydroxyproline residues, meaning that less than 2% of the aminoacid residues in the gelatin-like protein are hydroxyproline residues, preferably less than 1%, more preferably no hydroxyproline residues are present. The amount of hydroxyprolines can be determined by any standard aminoacid analysis method like, for example, described in HP AminoQuant Series II, operators handbook, 1990, Hewlett-Packard GmbH, Federal Republic of Germany, Waldbronn Analytical Division, HP Part No. 01090-90025.

"Free of triple helix" structure refers to essentially the absence of the positive peak characteristic of the collagen triple helix in a circular dichroism spectrum. Circular dichroism spectrometry can be carried out as described in Werten et al. (2001, Protein Engineering 14:447-454).

Although short intervening amino acids or stretches of amino acids may be present between the repeat units, it is preferred that the repeats are essentially free of intervening amino acids, whereby "essentially free" means that less than 5, 4, 3, 2 or 1, most preferably 0 intervening amino acids are present between monomers. The multimer may comprise additional amino acids at one or both ends, e.g at the N- and/or C-terminal. For example, 1, 2, 3, 6, 9, 12, 15 or more amino acids may be present. These may be in the form of GXY triads.

In order to facilitate multimer construction, the multimeric protein may comprise N-terminal and C-terminal amino acids that are not part of the repeating amino acid sequence.

The monomer unit may be a fragment of a natural collagen protein (such as human type I, II or III collagen proteins, e.g. Col1A1, Col3A1, etc.) but is preferably a synthetic sequence, not occurring in nature.

In one embodiment the repeat with molecular weight below 70 kDa comprises or consists of SEQ ID NO: 4 (P4 repeat unit) or an amino acid sequence essentially identical thereto.

The monomer and repeat nucleic acid sequences are preferably made using known molecular biology techniques, e.g. from *de novo* synthesis or by cloning fragments of natural collagen like proteins and optionally further DNA modification to encode the desired amino acids. Once a nucleic acid sequence encoding the monomer is made, standard cloning techniques can be used to repeat this nucleic acid sequence in a linear fashion in order to generate a nucleic acid sequence encoding the high molecular weight protein (An example can be found in Werten et al. (2001, Protein Engineering 14:447-454). Due to the degeneracy of the genetic code, obviously different nucleic acid molecules can encode the same amino acid sequence. The codon usage of the nucleic acid sequence is preferably adapted to the codon usage of genes which are highly expressed in the host (see Sreekrishna and Kropp, 1996, Nonconventional yeasts in biotechnology. A handbook. Springer, Berlin, p203-253).

Thus, the nucleic acid sequence encoding the monomer is preferably repeated consecutively, to form a nucleic acid sequence encoding a high molecular weight multimer, which can then be produced in a recombinant microorganism host as described herein below.

Preferred multimers are multimers of P repeat unit (monomer) and/or P4 repeat described above, or variants of these. Most preferably, the same monomer and/or repeat unit is repeated to form the high molecular weight polypeptide. In one embodiment P 8-mer, P 12-mer and P 16-mer are provided herein, as depicted in SEQ ID NO: 5, 6, and 7, respectively, as well as variants thereof. In one embodiment the multimer recombinant gelatins according to the present invention, e.g. SEQ ID NO 5, 6 and 7 are preceded by a glycine-proline-proline (GPP) triplet and extended with two glycine residues (GG) at the carboxy-terminus. Thus recombinant gelatins according to the present invention include GPP((SEQ ID NO: 3))ₓGG, wherein x is an integer selected of 5 and higher, preferably x is 8 or 12 or 16. Respectively these sequences are SEQ ID NO: 8, 9 and 10 and are preferred embodiments according to the present invention. The high molecular weight proteins preferably have a calculated molecular weight of at least about 40, 50, 60, 70, 80, 90, 100, 120, 140, 180, 220, 260 up to about 300 or more kiloDaltons (kDa). The molecular weight can be calculated using computer programs such as EmbossWin pepstats. SDS-PAGE measured molecular weights may not allow a correct size estimation to be made. Preferably when referring to "a polypeptide" a plurality of polypeptides, of the same amino acid sequence and molecular weight are meant, i.e. a "homogenous" composition of proteins is referred to, unless stated otherwise herein. In certain embodiments also defined mixtures of two, three or more high molecular weight proteins are provided (see below).

### Methods of producing high molecular weight polypeptides according to the invention

It was surprisingly found that by repeating monomer units, very high yields of high molecular weight proteins could be obtained. Without being bound by any theory, it is therefore thought that a direct correlation exists between the yield and molecular weight of multimeric proteins.

The high molecular weight multimer gelatines according to the invention can be produced by recombinant methods as disclosed in EP-A-0926543, EP-A-1014176 or WO01/34646. Also for enablement of the production and purification of gelatines of the invention reference is made to the examples in EP-A-0926543 and EP-A-1014176.

The polypeptides can be produced by expression of nucleic acid sequence encoding such polypeptides by a suitable micro-organism. The process can suitably be carried out with a fungal cell or a yeast cell. Suitably the host cell is a high expression host cell like *Hansenula, Axula, Trichoderma, Aspergillus, Penicillium, Saccharomyces, Kluyveromyces, Neurospora* or *Pichia.* Fungal and yeast cells are preferred to bacteria as they are less susceptible to improper expression of repetitive sequences. Most preferably the host will not have a high level of proteases that attack the collagen structure expressed. In this respect *Pichia* or *Hansenula* offers an example of a very suitable expression system. Use of *Pichia pastoris* as an expression system is disclosed in EP-A-0926543 and EP-A-1014176. In one embodiment the micro-organism is free of active post-translational processing mechanism such as in particular hydroxylation of proline and also hydroxylation of lysine. In another embodiment the host system has an endogenic proline hydroxylation activity by which the recombinant gelatine is hydroxylated. The selection of a suitable host cell from known industrial enzyme producing fungal host cells specifically yeast cells on the basis of the required parameters described herein rendering the host cell suitable for expression of recombinant gelatine-like proteins suitable in compositions according to the invention in combination with knowledge regarding the host cells and the sequence to be expressed will be possible by a person skilled in the art.

Also mutant host strains may be used, e.g. strains deficient in one or more proteolytic enzymes, although this is not necessary according to the present invention, as the recombinant polypeptides are highly stable and resistant to proteolysis.

In one embodiment a method for producing a high molecular weight multimer polypeptide, having a calculated molecular weight of at least 40kDa, is provided comprising the steps of:
- generating an expression vector comprising a promoter operably linked to a nucleic acid sequence, with the codon usage of genes which are highly expressed in the intended yeast host, encoding a multimer polypeptide as described herein above;
- transforming a yeast species, preferably *Pichia pastoris,* with said expression vector;
- culturing said transformed yeast host under suitable conditions for producing said polypeptide;
optionally purifying said polypeptide from the culture medium and/or the host cells;
wherein said high molecular weight polypeptide is produced at a level of at least 10g/l culture broth, preferably at least 12 g/l, more preferably at least 14 g/l.

### Compositions, products and uses according to the invention

In one embodiment the present inventions concerns a composition comprising at least one multimer polypeptide according to the present invention. In one embodiment the composition is a pharmaceutical composition or a nutritional- or nutraceutical composition. For example the present multimers can be used as a plasma expander in blood substitute liquids. Also the present multimers can constitute or be comprised in a matrix for controlled drug release. Also the invention further provides use of a such controlled release composition for the preparation of a medicament for the treatment of pain, cancer therapy, cardiovascular diseases, myocardial repair, angiogenesis, bone repair and regeneration, wound treatment, neural stimulation/therapy or diabetics.

In another embodiment, the present invention concerns a solid support comprising at least one multimer polypeptide according to the present invention. In one embodiment the solid support is a medical device, e.g. a stent, a cell support, or a dermal filler and the like.

A cell support comprising a multimer according to the present invention may for example be selected from the group consisting of
1) a cell-culture support, such as a core bead (e.g. a microcarrier bead) or a Petri dish or the like, coated with one or more multimer polypeptides according to the present invention;
2) an implant or transplant device (such as hip-, dental-, or other implants, etc.) coated with one or more of the multimer polypeptides according to the present invention,
3) a scaffold or matrix for tissue engineering, such as artificial skin matrix material, coated with one or more multimer polypeptides according to the present invention;
4) a wound healing product coated with one or more multimer polypeptides according to the present invention
5) a tissue adhesive comprising or consisting of one or more multimer polypeptides according to the present invention.

Also the present recombinant proteins are highly useful in photographic applications, e,g. as protective colloid in silver halide emulsions. Also in one embodiment the present invention concerns a silver halide emulsions comprising a gelatin according to the present invention.

### EXAMPLES

The construction of pPIC9-P4, the vector comprising the gene for the tetramer of P, has been described in detail in Werten et al. (2001, Protein Engineering 14:447-454), which is incorporated by reference herein.

The BglII-Not fragment from pPIC9-P4 containing the AOX1 promoter and the gene for P4 was subcloned from pPIC9-P4 into pPICZ A digested with the same enzymes to yield pPICZ-P4. The DraIII site in the Zeocin resistance gene from pPICZ-P4 was removed by site-directed mutagenesis to render the DraIII site in the gene for P4 unique. The HindIII- PflMI fragment containing the P4 gene from pPICZ-P4 was subcloned into pPICZ-P4 digested with DraIII and HindIII.

This resulted in the formation of plasmid pPICZ-P8. Plasmid pPICZ-P12 was generated by subcloning the HindIII-PflMI fragment from pPICZ-P8 into pPICZ-P4-digested with HindIII and DraIII. By analogy, pPICZ-P16 was generated by subcloning the HindIII-PflMI fragment from pPICZ-P8 into pPICZ-P8-digested with HindIII and DraIII.

The plasmids pPICZ-P4, pPICZ-P8, pPICZ-P12 and pPICZ-P16 were linearized with PmeI and transformed into *P. pastoris* X-33. Multicopy integrants were selected on 1.0 and 1.5 mg/ml of Zeocin. Manufacturer's (Invitrogen) protocols were followed.

Representative strains resulting from these transformations were grown in high-density cell cultures under standard fermentation conditions (at a pH of about 4), and the yield of the relevant gelatins in the supernatants was determined using UPLC (using BSA as a standard).

Yields obtained were:
P4 7-9 g/l
P8 14.19 - 15.89 g/l
P12 14.23 - 18.41 g/l

## Claims

1. A multimer polypeptide consisting of or comprising at least 5 consecutive repeat units of a monomer polypeptide unit, wherein said monomer consists of or comprises the amino acid sequence SEQ ID NO: 3.

2. The multimer polypeptide according to claim 1, wherein said multimer polypeptide comprises the amino acid sequence of SEQ ID NO: 5, 6, or 7

3. The multimer polypeptide according to any one of the preceding claims, wherein said multimer polypeptide consists of the amino acid sequence of SEQ ID NO: 8, 9, or 10.

4. A composition comprising at least one multimer polypeptide according to any one of claims 1-3.

5. The composition according to claim 4, wherein said composition is a pharmaceutical composition or a nutritional- or nutraceutical composition.

6. A solid support comprising at least one multimer polypeptide according to any one of claims 1-3.

7. The solid support according to claim 6, wherein the support is a medical device, a cell support or a dermal filler.

8. A method for producing a high molecular weight multimer polypeptide having a calculated molecular weight of at least 40 kDa, comprising the steps of:
- generating an expression vector comprising a promoter operably linked to a nucleic acid sequence with the codon usage of genes which are highly expressed in the intended yeast host, encoding a multimer polypeptide according to any one of claims 1-3;
- transforming a yeast species with said expression vector;
- culturing said transformed yeast host under suitable conditions for producing said polypeptide;
- optionally purifying said polypeptide from the culture medium and/or the host cells, wherein said high molecular weight polypeptide is produced at a level of at least 10 g/l culture broth.

9. A method according to claim 8 wherein the yeast species is *Pichia pastoris.*

10. The method according to either claim 8 or claim 9, wherein said multimer polypeptide comprises SEQ ID NO: 5, 6 or 7 or consists of SEQ ID NO: 8, 9, or 10.

## Patentansprüche

1. Multimer-Polypeptid, bestehend aus oder umfassend wenigstens 5 aufeinander folgende Wiederholungseinheiten einer Monomer-Polypeptideinheit, wobei das Monomer aus der Aminosäuresequenz SEQ ID NO: 3 besteht oder diese umfasst.

2. Multimer-Polypeptid nach Anspruch 1, wobei das Multimer-Polypeptid die Aminosäuresequenz der SEQ ID NO: 5, 6 oder 7 umfasst.

3. Multimer-Polypeptid nach einem der vorhergehenden Ansprüche, wobei das Multimer-Polypeptid aus der Aminosäuresequenz der SEQ ID NO: 8, 9 oder 10 besteht.

4. Zusammensetzung, umfassend wenigstens ein Multimer-Polypeptid nach einem der Ansprüche 1-3.

5. Zusammensetzung nach Anspruch 4, wobei es sich um eine pharmazeutische Zusammensetzung oder eine Ernährungs- oder nutrazeutische Zusammensetzung handelt.

6. Fester Träger, umfassend wenigstens ein Multimer-Polypeptid nach einem der Ansprüche 1-3.

7. Fester Träger nach Anspruch 6, wobei es sich um eine medizinische Vorrichtung, einen Zellträger oder einen Hautfüllstoff handelt.

8. Verfahren zur Herstellung eines hochmolekularen Multimer-Polypeptids mit einem berechneten Molekulargewicht von wenigstens 40 kDa, wobei man die folgenden Schritte durchführt:
- Erzeugen eines einen Promotor in operativer Verknüpfung mit einer Nukleinsäuresequenz mit der Codonverwendung von Genen, die in dem vorgesehenen Hefewirt stark exprimiert werden, die für ein Multimer-Polypeptid nach einem der Ansprüche 1-3 codiert, umfassenden Expressionsvektors;
- Transformieren einer Hefespezies mit dem Expressionsvektor;
- Kultivieren des transformierten Hefewirts unter geeigneten Bedingungen für die Herstellung des Polypeptids;
- gegebenenfalls Aufreinigen des Polypeptids aus dem Kulturmedium und/oder den Wirtszellen, wobei das hochmolekulare Polypeptid in einer Konzentration von wenigstens 10 g/l Kulturbrühe produziert wird.

9. Verfahren nach Anspruch 8, wobei es sich bei der Hefespezies um *Pichia pastoris* handelt.

10. Verfahren nach Anspruch 8 oder Anspruch 9, wobei das Multimer-Polypeptid SEQ ID NO: 5, 6 oder 7 umfasst oder aus SEQ ID NO: 8, 9 oder 10 besteht.

## Revendications

1. Polypeptide multimérique constitué par ou comprenant au moins 5 unités de répétitions consécutives d'une unité de polypeptide monomérique, dans lequel ledit monomère est constitué par ou comprend la séquence d'acides aminés SEQ ID n° : 3.

2. Polypeptide multimérique selon la revendication 1, dans laquelle ledit polypeptide multimérique comprend la séquence d'acides aminés de SEQ ID n° : 5, 6 ou 7.

3. Polypeptide multimérique selon l'une quelconque des revendications précédentes, dans laquelle ledit polypeptide multimérique est constitué par la séquence d'acides aminés de SEQ ID n° : 8, 9 ou 10.

4. Composition comprenant au moins un polypeptide multimérique selon l'une quelconque des revendications 1 à 3.

5. Composition selon la revendication 4, dans laquelle ladite composition est une composition pharmaceutique ou une composition nutritionnelle ou nutraceutique.

6. Support solide comprenant au moins un polypeptide multimérique selon l'une quelconque des revendications 1 à 3.

7. Support solide selon la revendication 6, dans laquelle le support est un dispositif médical, un support cellulaire ou une substance de comblement dermique.

8. Procédé de production d'un polypeptide multimérique de poids moléculaire élevé, ayant un poids moléculaire calculé d'au moins 40 kDa, comprenant les étapes consistant à :
- générer un vecteur d'expression comprenant un promoteur qui est lié, de manière opérationnelle, à une séquence d'acide nucléique, avec l'utilisation de codons de gènes qui sont fortement exprimés dans la levure hôte prévue, codant pour un polypeptide multimérique selon l'une quelconque des revendications 1 à 3 ;
- transformer une espèce de levure avec ledit vecteur d'expression ;
- cultiver ladite levure hôte transformée, dans des conditions appropriées pour produire ledit polypeptide ;
- en option, purifier ledit polypeptide à partir du milieu de culture et/ou des cellules hôtes, dans lesquels ledit polypeptide de poids moléculaire élevé est produit à un taux d'au moins 10 g/l de bouillon de culture.

9. Procédé selon la revendication 8, dans laquelle la levure est de l'espèce *Pichia pastoris.*

10. Procédé selon la revendication 8 ou la revendication 9, dans laquelle ledit polypeptide multimérique comprend les SEQ ID n° : 5, 6 ou 7 ou est constitué par les SEQ ID n° : 8, 9 ou 10.
